# EUROPEAN PATENT APPLICATION

(11) **EP 2 048 155 A1**
(43) Date of publication of application: **15.04.2009**
(21) Application number: 07075864.4
(22) Date of filing: 02.10.2007
(51) Int. Cl.: C07K 11/00, C07K 14/195, C12N 15/31, C12N 9/10, C12P 21/04, C12N 1/15, C12N 1/21

(54) **Method and means for the production of pharmaceutically active natural products**

(71) Applicant: Humboldt-Universität zu Berlin, D-10099 Berlin (DE)
(72) Inventor: Dittmann, Elke, Prof.Dr., 10407 Berlin (DE); Ziemert, Nadine, 12621 Berlin (DE)
(74) Representative: Maikowski, Michael

(57) **Abstract**

Summary

Means and a method for the biosynthesis of depsipeptide natural compounds are provided. According to the invention, a nucleic acid molecule comprises a nucleic acid sequence, which encodes a precursor peptide sequence of between 5 and 50 amino acids that is susceptible to post-translational modification, and one or more nucleic acid sequences encoding enzymes for post-translational modification, of which at least one is an ATP-grasp-like ligase enzyme. The nucleic acid molecule can be introduced to cells, preferably prokaryotic cells such as E. coli or Bacillus. A method for the production of depsipeptide natural compounds comprises growing such cells, harvesting and isolating the resultant product.

SEQUENCE LISTING

<110>  Humboldt-Universität Berlin

<120>  Method and Means for the Production of Pharmaceutically

<130>  IPA139EP

<160>  27

<170>  PatentIn version 3.3

<210>  1
<211>  153
<212>  DNA
<213>  Microcystis aeroginosa

<400>  1
atggcatatc ccaacgatca acaaggtaaa gcacttcctt tctttgctcg tttcttgtcc      60

gtaagcaaag aggaatcttc catcaagtct ccttcccctg agcctacctt cgggaccacc     120

ttgaaatacc cttctgactg ggaagagtat taa                                  153


<210>  2
<211>  978
<212>  DNA
<213>  Microcystis aeroginosa

<400>  2
   atgaaagaat cgcctaaagt tgttttattg ttgacccata gcggcgattt tttcacgatt      60
   gaccgcgtgg ctgaggcgat agaaaaaaaa ggagccacac cttttcgctt agataccgat     120
   aaatttccct tagaggttca attagcagcc caatttaatg ggaaaaaaag tttttatcaa     180
   ttaacctata accatcaatc tattgatagt gaacaggtgc aatctgtttg gacaagacgt     240
   atttggcaac ctgaaataac aggagattta gatcctcagt ttcgggaggc ttgtgtgcga     300
   gaatcccaga cgactttggc tggtttttgg gatagtttaa ggtcagcgcg ttggttagat     360
   aatttagcgc aaattgagag ggctaaaaat aagttattac aacttcgttt ggcctcagaa     420
   gttggcttga ttatcccccc cactttagtg accaataatc ctgatgctgc ccgggagttt     480
   ttttcccagg ttcagggaag aatggtgagt aaattgttaa ctgcgatcgc ccgaagtatg     540
   gagtcgccgg aattttttct ttataccagt agggtgaaag cagaagacct tgaggaagcg     600
   gaatctttac gttattgtcc catggttttt caagcggaaa ttcccaaaca attagaattg     660
   agagttgtcg tggttaatgg acagacattt gtgggagctt tagaatcttc tcagtataat     720
   aattctgccg tagattggcg aagaccgggt attgatcctg gtgcttggca acatcatact     780
   ttacccgatt cactattgca gcaacttcag atttttatgg ctaatttagg gttgaatttt     840
   ggggcgtttg attttatctt aactcctggg ggagaatacg tttttttaga agttaatccg     900
   ggtggtgagt ggggaatgtt agaacgggat ttagatttac ctatttctaa tgcgatcgct     960
   gattttttag tatgctaa                                                  978


<210>  3
<211>  849

<212>  DNA
<213>  Microcystis aeroginosa

<400>  3
ttgatcttta ctcaggcggt caaaaaggcg gaaattatta ccgatggaga ccaaaaatta      60

gagctaaaag aagtttctgc tgtctggtat cgacgcatga gatacggact aaaattaccc     120

gatgggatgg atagtcaatt tcgcgaagct tctcttaagg aatgtcggtt aagtattcga     180

ggaatgattg ctagtttatc tggctttcat cttgatccaa ttgctaaggt agatcatgcc     240

aatcataaac aattgcagtt acaagtagcg caacaattag gtttattaat tccggggact     300

ttaacttcta ataatcctga agctgtcaag caatttgctc aggagtttga agcaacggga     360

attgtgacta aaatgctttc tcaatttgct atttatggag acaagcaaga ggaaatggtt     420

gtttttacca gtcctgttac aaaggaagac ctagataatt tggaaggttt gcaattttgt     480

cccatgactt ttcaggaaaa cattcctaaa gctttggaat tacgcatcac tattgtcggt     540

gaacaaatat ttacggcggc gattaattcc caacaattag acggtgctat ctacgattgg     600

cgaaaagagg gaagagcgct ccatcaacaa tggcaaccct acgatttacc gaaaactatt     660

gaaaaacaac tactagaatt agtgaaatat ttcggtctca attatggtgc aattgatatg     720

attgtcacac cagatgaacg ttatatcttt ttagaaatta atcccgttgg cgagtttttc     780

tggctagaac tttatcctcc ttattttcct atctcccagg cgatcgctga agtcctcgtc     840

aactcataa                                                             849

<210>  4
<211>  534
<212>  DNA
<213>  Microcystis aeroginosa

<400>  4
atgaaagcac tggaaaaact ggtgacggaa cgattatatt tagtcccctt aaagcttgaa      60

cttctcaagg ccgcaattat aggtatcgat gaattagcaa caattttggg ggtaaaagtt     120

gccgatgact gggccgaaca agaattgatt caagctttcc ctgatattgc gaatattttg     180

ctgcaattcc ccttacaaaa tgaatgggga tgggggagtt taattattca tcaagcagaa     240

aatactctta ttggtcatgt gatgatcaaa attattcctg atgtcacagg tttacccaca     300

gatgctgtag aaattggcta tcaagtagca ccgttatatc gacgacaagg ctacgcatct     360

gaggcgacaa aagcaatgat ggactggaca ctttctcaac ctggtatgca aacagtgacc     420

gctggctgcg ctcccgataa tatagcttca aagcgagttt tagaaaaaat aggcatggat     480

ctgatagaag cgcgagagaa agtcttgatc tggaaattaa gtagggtttg ctga           534

<210>  5
<211>  9934
<212>  DNA
<213>  Microcystis aeroginosa

<220>
<221>  misc_feature

<222>  (9881)..(9881)
<223>  n is a, c, g, or t

<400>  5
tcatcccgaa aagacaggta caattaaagt cggcgatcct attaccgtaa tctagggaaa      60

tggggttatc agttatcagt tatcagttat cagatgtgag ttttcagttc actgattaag     120

tacctaagca aaattaatta cacatatcta accccgctct tgccttttgc cttttgcctc     180

ttgcctatct tcactaggaa attaattttg cacgactact tactgttcac tgttcacagc     240

aaaaaactcc ccactcccca ctgataactg tgccggcatc aaggttaaga taaataacca     300

taagaacaat acttgattag agacactgaa accatgcccc ttgataccgc atcgatttta     360

gaagtattac gtcccgttca agatccagaa ctgcaaaaaa gcctagtgga attgaacatg     420

atccgcaatg tggcgatcga tggcggaaaa gttagcttta ccctagtttt gaccactcct     480

gcctgtcctt tgcgcgaatt tatcgtggaa gactgccaaa aagccgttaa acagctgccc     540

ggggtggaaa gtgtcgccgt ggaggtaact gccgaaaccc cgcaacaaaa agctttaccc     600

gatcgccaag gtgtggaggg ggttaaaaat attatcgccg tttccagtgg taagggcggt     660

gtgggaaaaa gtaccgtggc tgtcaatatc gccgtcgcct tggctcattt aggggcaaaa     720

gtggggctat tagacgcgga catctacggc cccaacgccc cgacaatgtt gggcttaaat     780

gacgcacaag tgaccgtaca gggcgcaaat ggcgagatat tggaacccgc ttttaatcat     840

ggcatcaaaa tggtttccat gggctttttg atcaaccccg atcaaccggt aatctggcgc     900

ggtccgatgt taaacggcat tatccgtcag tttctctatc aagtcaattg gggcgattta     960

gactatttaa tcgtcgatat gcctcccggt actggggatg cacaattaac tttaattcaa    1020

tcggtccccc tagccggtgc ggtgattgtc accactcccc agacggtttc tttaattgat    1080

gcgcgtcggg gtttaaaaat gttccaacag ctaggggcgc gggttttggg tatcgtggaa    1140

aatatgagtt attttatccc tcccgatcag cccgatcgct cctatgatct atttgggtcc    1200

ggtggtgggg aaaaaacctc gcaagaattg ggcattcctt tactgggatg tgtgcctctg    1260

gagatagccc tccgggaggg cggtgatact ggtgtaccag tagttttagg gcaaccagaa    1320

tcggcctcag ctaaagccct gatagcgatc gctcgtcagg tggcggcgaa agtatccgtg    1380

gcagcctata gttagggttt gcggcaaaaa gttttcccta ggggcagggt gtggggtgta    1440

gggtgtaggg ttttacccat tttcagggaa aaagtccagg gattttcccc cccgatcact    1500

ccaagggccg gtacttttgg atttcaaaaa ggtctaaaag tattatcgaa caatgttttt    1560

agatttattg agcaaaccct agttagttag taaatttggg gagagaaatg gtgagaagac    1620

agtcccttat tagtaggaaa ctaagcggtt ttagtgagca gtttagcttc attttccggg    1680

atatagccca aatggatcgg cttttgttcg ttttggtgac gggtttaacc gttttcggtg    1740

gtttaatgat tcgtagtgcc gaaagacata ccactgctct cgattggtgg caacactggt    1800

tattcggatg cgtgggagtg gcgatcgctt tatttctggc ccgttgtcgt tacgaaagtc    1860

tgctgaaatg gcactggtta acctatcttc tcaccaatct ctccctaatc gcggtaatta    1920

tcctaggcgt gacggcgaat ggggcgcaaa gttggattaa tatcggcagt tttaacgtgc 1980
aaccgtcgga atttgccaag gtaggtttaa ttatcaccct agcagccctc ctacatcatc 2040
gtcccgccga taatcttttt gcgatcgctc gtgtctttgc cgtcactgcc atcccctggg 2100
tgttaattat ggcgcagccg gacctgggga caggattagt ctttggggca attaccctag 2160
gcatgattta ctgggctaat gcgaaactgc cttggatgat tatcctgtta tctcccatcg 2220
catcggtttt tctgtttaat ttactttttc ctgcttggat tgttttagca attatcatcg 2280
ctgtgcttgc ttggtttacc cttccctacc gttttttatc tactttcatc gtggtggcaa 2340
ctaatctggc agtgggtaaa ttaggcgagg ttttttgggg tttattaaaa gaatatcaaa 2400
aagatcgttt aaccctattt cttcacccag aaaagaatcc tttgggggga ggttatcaat 2460
taattcaatc ccgcattgcc atcggatcag gagaattatt gggacgggga ttacaccaag 2520
gcacccaaac ccaattaaat tttattcccg aacaacacac cgattttatc ttctcggtag 2580
tcggggaaga actcggtttt gttggcagta ttctagtttt aatagctttc tggttagttt 2640
gttggcgttt gttagtcatt gctaatactg ctaaagaaaa cttcggttct ttaatagcga 2700
tcggtgttct ctcgatgatc gcttttcagg caattcttaa tattagcatg accgtgggat 2760
tagccccgat tacagggatt cctttacctt ggttaagtta tggacgttca tctttattaa 2820
ccaattttat cgccttgggt ttagtggaat ccgtggctaa ttatcgcccc cgtaagcgtt 2880
tatactaagt agttgtgcag ctatcagtta tcagtgatca gttatcagat ttgagtttta 2940
agtgtggagt attaaatccc actttccgca cttcttaaca ttcaattgat gatttttact 3000
aatatattcc ttccaaaccc ttgccagtaa gaattataac tacaatgatg gattttgatc 3060
aaggtgaaga attgtaaatt tttttctcag aaaaaatcaa ttattgaaaa tgttctcaat 3120
agtagtttct acaatcaagg tttttcgtta aaacatcttg gggaatgtaa aaatatataa 3180
aagtatatga agatgtgcgg aatgtcggat ttagcggaaa tttttcttaa atttttctta 3240
agtgcttgac attggccaga ttagtgctta tgatgtttcc catcacccaa ataaaagcaa 3300
attatttgca caatcccaat tctgaaagcc atgcacagca aagctttcag gggttattgc 3360
aggtggttag ttttatctga gtgattaaac ctattcactc gaaattacca gaggaataac 3420
tatggcatat cccaacgatc aacaaggtaa agcacttcct ttctttgctc gtttcttgtc 3480
cgtaagcaaa gaggaatctt ccatcaagtc tccttcccct gagcctacct tcgggaccac 3540
cttgaaatac ccttctgact gggaagagta ttaaactcag atcacttgag taattacagc 3600
acttttcttg attacaccgt acagacaaaa agctaaaagc tccactaagc aaggctcgca 3660
gccaaaaaac tatacgttga gatcctgaga agtgctgtca actaaattta gtaattactc 3720
aagaaaaaac caatactgca ataagtcaag caaaagcaag actttttgca gcattttctt 3780
gagattagga tcaataaaga ccgacccaaa agccttaaga tgggttaaaa tgtaaggact 3840
tgcaattgac atgaaagaat cgcctaaagt tgttttattg ttgacccata gcggcgattt 3900
tttcacgatt gaccgcgtgg ctgaggcgat agaaaaaaaa ggagccacac cttttcgctt 3960

agataccgat aaatttccct tagaggttca attagcagcc caatttaatg ggaaaaaaag 4020
tttttatcaa ttaacctata accatcaatc tattgatagt gaacaggtgc aatctgtttg 4080
gacaagacgt atttggcaac ctgaaataac aggagattta gatcctcagt ttcgggaggc 4140
ttgtgtgcga gaatcccaga cgactttggc tggtttttgg gatagtttaa ggtcagcgcg 4200
ttggttagat aatttagcgc aaattgagag ggctaaaaat aagttattac aacttcgttt 4260
ggcctcagaa gttggcttga ttatcccccc cactttagtg accaataatc ctgatgctgc 4320
ccgggagttt ttttcccagg ttcagggaag aatggtgagt aaattgttaa ctgcgatcgc 4380
ccgaagtatg gagtcgccgg aattttttct ttataccagt agggtgaaag cagaagacct 4440
tgaggaagcg gaatctttac gttattgtcc catggttttt caagcggaaa ttcccaaaca 4500
attagaattg agagttgtcg tggttaatgg acagacattt gtgggagctt tagaatcttc 4560
tcagtataat aattctgccg tagattggcg aagaccgggt attgatcctg gtgcttggca 4620
acatcatact ttacccgatt cactattgca gcaacttcag atttttatgg ctaatttagg 4680
gttgaatttt ggggcgtttg attttatctt aactcctggg ggagaatacg tttttttaga 4740
agttaatccg ggtggtgagt ggggaatgtt agaacgggat ttagatttac ctatttctaa 4800
tgcgatcgct gattttttag tatgctaaaa atcaaaccaa aacctaatca ggattttgag 4860
gattttagga ttatcaagat tctcaatcca aaatctaatc aggattctca atcccaaatc 4920
atcaaggaaa tctatgaccg ttttaattgt tacttttagc catgataatg aaagtattcc 4980
tctggtaatc aaagccatag aaacgatggg taaaaaagcc ttccgttttg ttgaggattt 5040
taggattatc aagattctca atccaaaatc taatcaggat tctcaatccc aaatcatcaa 5100
ggaaatctat gaccgtttta attgttactt ttagccatga taatgaaagt attcctctgg 5160
taatcaaagc catagaaacg atgggtaaaa aagccttccg ttttgttgag gattttagga 5220
ttatcaagat tctcaatcca aaatctaatc aggattctca atcccaaatc atcaaggaaa 5280
tctatgaccg ttttaattgt tacttttagc cacgataatg aaagtattcc tctggtaatc 5340
aaagccatag aaacgatggg gaaaaaagcc ttccgttttg ataccgatcg cttccctaca 5400
gaggtgaaag ttgatcttta ctcaggcggt caaaaaggcg gaaattatta ccgatggaga 5460
ccaaaaatta gagctaaaag aagtttctgc tgtctggtat cgacgcatga gatacggact 5520
aaaattaccc gatgggatgg atagtcaatt tcgcgaagct tctcttaagg aatgtcggtt 5580
aagtattcga ggaatgattg ctagtttatc tggctttcat cttgatccaa ttgctaaggt 5640
agatcatgcc aatcataaac aattgcagtt acaagtagcg caacaattag gtttattaat 5700
tccggggact ttaacttcta ataatcctga agctgtcaag caatttgctc aggagtttga 5760
agcaacggga attgtgacta aaatgctttc tcaatttgct atttatggag acaagcaaga 5820
ggaaatggtt gtttttacca gtcctgttac aaaggaagac ctagataatt tggaaggttt 5880
gcaattttgt cccatgactt ttcaggaaaa cattcctaaa gctttggaat tacgcatcac 5940
tattgtcggt gaacaaatat ttacggcggc gattaattcc caacaattag acggtgctat 6000

ctacgattgg cgaaaagagg gaagagcgct ccatcaacaa tggcaaccct acgatttacc 6060
gaaaactatt gaaaaacaac tactagaatt agtgaaatat ttcggtctca attatggtgc 6120
aattgatatg attgtcacac cagatgaacg ttatatcttt ttagaaatta atcccgttgg 6180
cgagtttttc tggctagaac tttatcctcc ttattttcct atctcccagg cgatcgctga 6240
agtcctcgtc aactcataaa ccaacaaata cagagccaga gtattttatc gcatcgacga 6300
ggctctgact ccagatttcc cccccgacta ctgacttgaa agagggtgta gggtgtgggg 6360
tgtggggtgt agggtgtggg aagtggggaa gtggggagtt ttctcagtga actgataact 6420
gataactgat aactgactcc taaccccacc aacaaacttt ttcagcaaac cctacttaat 6480
ttccagatca agactttctc tcgcgcttct atcagatcca tgcctatttt ttctaaaact 6540
cgctttgaag ctatattatc gggagcgcag ccagcggtca ctgtttgcat accaggttga 6600
gaaagtgtcc agtccatcat tgcttttgtc gcctcagatg cgtagccttg tcgtcgatat 6660
aacggtgcta cttgatagcc aatttctaca gcatctgtgg gtaaacctgt gacatcagga 6720
ataattttga tcatcacatg accaataaga gtattttctg cttgatgaat aattaaactc 6780
ccccatcccc attcattttg taaggggaat tgcagcaaaa tattcgcaat atcagggaaa 6840
gcttgaatca attcttgttc ggcccagtca tcggcaactt ttacccccaa aattgttgct 6900
aattcatcga tacctataat tgcggccttg agaagttcaa gctttaaggg gactaaatat 6960
aatcgttccg tcaccagttt ttccagtgct ttcatttgat aaaactccta taccagttat 7020
cagttatcag ttatcagtta gtagcttgtt aatcaactta cccagcaaaa acctgattct 7080
ctagttctcc tgagagggga aagtctgaat actctgtctc aatttcaatc tgtttgtcac 7140
cccattagct atattctcac ccattttaag gtttttgggt ctttatcgta acgataatta 7200
aaaccatctt tggcagtaat aattttacct aaactggcac gactccggac gttacttaaa 7260
gaataatcag ttagggactg catttgttga tgggacaaac cttctgctat ttcagaaact 7320
ccggcaattt ccgcatcatg ctcagtttta acctcagaca aagtctctat ttttaccgac 7380
gaaaaatttt ctgatttttt tgacatcatc actaggttat tcactttttt gcgctgataa 7440
tcttctacag ttgacagctg ccaatgggaa ttttctgtta actctaagac ccatcgatga 7500
taattaaata aactttcccg atgaccaaca ctaataaagg tggtttcact ttcctgtaat 7560
tgctgatata agctggcttc attggccaaa tctaaagcgc tggttgcttc atctaagata 7620
gtaaaagtgg gtttagtgat taatagtcga gcaaaagcta atctttgttg ttctccgagg 7680
gataaaatct gttcccaatt gacttgttta tccaaggtat ttttgtcagt taataaatgt 7740
tgcaggttga ctttttttaa gatgttttct aattcttcat cgctcacctg ctcattggta 7800
tggggataaa gtaattgctg gcgtaaagtg ccaaggataa tataaggacg ttggggtaaa 7860
aataacattt tagctaaagg aggacgaact aaacgaccac tacctgcttt ccataaaccg 7920
gcgattgctc gtaacaagga actttttcct cgaccactgg ggccaataat cagtaagctt 7980
tctcccaaag ggacagaaac cgataaattt tccacaatta ccttttcata atcgggagtt 8040

tgtagggtaa aattttcaaa agctagacga ttatcttcca gggtggtaat acgacttaag 8100
tttttcggct cttcactggc aacttttaag gcatctgtca aggtagctaa ccgttcaata 8160
taacttgaaa ctttacccga tgttccccat tcagtaatta attgtcccaa ggcattagaa 8220
aaaaggaaac agcataaact cgcttgacta atttccccat aatcgatttc gtttttaata 8280
aacatcgggg tcaaaataaa cattgaaaat acactaatcg ctgactgata acctcgatta 8340
aaaagattat taaaccgttc ccaattgatc agtttttccg agctttgaat aacccgttga 8400
aaacggtctt caatgatttt ttcttcctgc tcttctccct gaaaaaaagc aattgattcg 8460
gcgtggttac gcacatgggt taaagcataa ttataatcag ctttatgatt aagctccgat 8520
tgattaattt tgtttaattc ttgagttaga taaattgcta tgaaatttcc tgcgattgta 8580
taaattatta gataaaccgc aatttgccga gaaattgtcc aaagaataat cgcaaaagta 8640
atcatttgca accctttttc cacgaaggta attaacaacc ttagggtcat ggtcgtaatt 8700
ggttcaattt cttgagataa tctttgatcg ggatttttta aagctgaggt aaaatcaatg 8760
ttataataag cgcgatcatt gagatatttt ttaacggttt gtttggttaa ccacttatac 8820
caatccaatg ccacttgacg acgaataaat tgagaaaaag caaataaccc cgttgttcct 8880
ataatcaaca aactggataa ccaaagcgta tttagatatt tagaaagatc tctatcttca 8940
ataataatat cgagtacata acgattccag tagctactaa aagcttctac cccgactgtt 9000
gccaagatac ttaaaaggat caaagctgac attccccaag aaatgatcac atctccaaag 9060
gcacgtccat ttaactccgt tggataccaa tagggttgag ccacaatttt taggctttcc 9120
caaaattgac caaatagtga aaagggatta ttggcggact ggtcggacac aacagggttt 9180
tccgttgctt gtttagtagt atattgaggc atctagatcc aattggcagt tcttttactg 9240
ttctcttgac aactcgctat catattgtag cggcagaatt acctcttgtt gtcttttgtt 9300
gacattttta tctagaagat gaaagccagc tatggtcaga aagctctgac ataactaaga 9360
ctacttgacc cacttttgtt actgtggcag tctagatgct gtttactggt cactgaaaaa 9420
ccccatctct caatctccgt gatcctcaaa atgattagct agtaggaatt tttgactggt 9480
attcaactct tgccaaagct ggttaatttt ctcgtaagct tccgtggagg taagctttcc 9540
cccactgtgt aaaccagcga taaaagagac tctcagggcg aattcctgca aattagcgtt 9600
aaaaactaaa ttttgtggcg tgaataaacc cctgtagctg gttttagggt agagaaagtt 9660
atctttagaa ttgaattttt ccattgttac cgacccccca gtataggata tctacattat 9720
tatatattta aacttttgtt aacttaggct taacctaaga atttaaacct gtccctggat 9780
tcttaagaga gtagctgctg aacttgatta gctgccgcta tcccagaagt taaggccgtt 9840
tctagggaag aatttctcga taaatctccc ccttgacacc natcaccaca ggcaactaga 9900
ggtaaaggac tatttaacac ttaaacaggg taca 9934

<210> 6
<211> 50
<212> PRT

<213>  Microcystis aeroginosa

<400>  6

Met Ala Tyr Pro Asn Asp Gln Gln Gly Lys Ala Leu Pro Phe Phe Ala
1               5                   10                  15

Arg Phe Leu Ser Val Ser Lys Glu Glu Ser Ser Ile Lys Ser Pro Ser
            20                  25                  30

Pro Glu Pro Thr Phe Gly Thr Thr Leu Lys Tyr Pro Ser Asp Trp Glu
        35                  40                  45

Glu Tyr
    50

<210>  7
<211>  325
<212>  PRT
<213>  Microcystis aeroginosa

<400>  7

Met Lys Glu Ser Pro Lys Val Val Leu Leu Leu Thr His Ser Gly Asp
1               5                   10                  15

Phe Phe Thr Ile Asp Arg Val Ala Glu Ala Ile Glu Lys Lys Gly Ala
            20                  25                  30

Thr Pro Phe Arg Leu Asp Thr Asp Lys Phe Pro Leu Glu Val Gln Leu
        35                  40                  45

Ala Ala Gln Phe Asn Gly Lys Lys Ser Phe Tyr Gln Leu Thr Tyr Asn
    50                  55                  60

His Gln Ser Ile Asp Ser Glu Gln Val Gln Ser Val Trp Thr Arg Arg
65                  70                  75                  80

Ile Trp Gln Pro Glu Ile Thr Gly Asp Leu Asp Pro Gln Phe Arg Glu
                85                  90                  95

Ala Cys Val Arg Glu Ser Gln Thr Thr Leu Ala Gly Phe Trp Asp Ser
            100                 105                 110

Leu Arg Ser Ala Arg Trp Leu Asp Asn Leu Ala Gln Ile Glu Arg Ala
        115                 120                 125

Lys Asn Lys Leu Leu Gln Leu Arg Leu Ala Ser Glu Val Gly Leu Ile
    130                 135                 140

Ile Pro Pro Thr Leu Val Thr Asn Asn Pro Asp Ala Ala Arg Glu Phe
145                 150                 155                 160

Phe Ser Gln Val Gln Gly Arg Met Val Ser Lys Leu Leu Thr Ala Ile

                165                 170                 175

Ala Arg Ser Met Glu Ser Pro Glu Phe Phe Leu Tyr Thr Ser Arg Val
            180                 185                 190

Lys Ala Glu Asp Leu Glu Glu Ala Glu Ser Leu Arg Tyr Cys Pro Met
        195                 200                 205

Val Phe Gln Ala Glu Ile Pro Lys Gln Leu Glu Leu Arg Val Val Val
    210                 215                 220

Val Asn Gly Gln Thr Phe Val Gly Ala Leu Glu Ser Ser Gln Tyr Asn
225                 230                 235                 240

Asn Ser Ala Val Asp Trp Arg Arg Pro Gly Ile Asp Pro Gly Ala Trp
                245                 250                 255

Gln His His Thr Leu Pro Asp Ser Leu Leu Gln Gln Leu Gln Ile Phe
            260                 265                 270

Met Ala Asn Leu Gly Leu Asn Phe Gly Ala Phe Asp Phe Ile Leu Thr
        275                 280                 285

Pro Gly Gly Glu Tyr Val Phe Leu Glu Val Asn Pro Gly Gly Glu Trp
    290                 295                 300

Gly Met Leu Glu Arg Asp Leu Asp Leu Pro Ile Ser Asn Ala Ile Ala
305                 310                 315                 320

Asp Phe Leu Val Cys
                325

<210> 8
<211> 282
<212> PRT
<213> Microcystis aeroginosa

<400> 8

Met Ile Phe Thr Gln Ala Val Lys Lys Ala Glu Ile Ile Thr Asp Gly
1               5                   10                  15

Asp Gln Lys Leu Glu Leu Lys Glu Val Ser Ala Val Trp Tyr Arg Arg
            20                  25                  30

Met Arg Tyr Gly Leu Lys Leu Pro Asp Gly Met Asp Ser Gln Phe Arg
        35                  40                  45

Glu Ala Ser Leu Lys Glu Cys Arg Leu Ser Ile Arg Gly Met Ile Ala
    50                  55                  60

Ser Leu Ser Gly Phe His Leu Asp Pro Ile Ala Lys Val Asp His Ala
65                  70                  75                  80

Asn His Lys Gln Leu Gln Leu Gln Val Ala Gln Gln Leu Gly Leu Leu
                85                  90                  95

Ile Pro Gly Thr Leu Thr Ser Asn Asn Pro Glu Ala Val Lys Gln Phe
            100                 105                 110

Ala Gln Glu Phe Glu Ala Thr Gly Ile Val Thr Lys Met Leu Ser Gln
        115                 120                 125

Phe Ala Ile Tyr Gly Asp Lys Gln Glu Glu Met Val Val Phe Thr Ser
    130                 135                 140

Pro Val Thr Lys Glu Asp Leu Asp Asn Leu Glu Gly Leu Gln Phe Cys
145                 150                 155                 160

Pro Met Thr Phe Gln Glu Asn Ile Pro Lys Ala Leu Glu Leu Arg Ile
                165                 170                 175

Thr Ile Val Gly Glu Gln Ile Phe Thr Ala Ala Ile Asn Ser Gln Gln
            180                 185                 190

Leu Asp Gly Ala Ile Tyr Asp Trp Arg Lys Glu Gly Arg Ala Leu His
        195                 200                 205

Gln Gln Trp Gln Pro Tyr Asp Leu Pro Lys Thr Ile Glu Lys Gln Leu
    210                 215                 220

Leu Glu Leu Val Lys Tyr Phe Gly Leu Asn Tyr Gly Ala Ile Asp Met
225                 230                 235                 240

Ile Val Thr Pro Asp Glu Arg Tyr Ile Phe Leu Glu Ile Asn Pro Val
                245                 250                 255

Gly Glu Phe Phe Trp Leu Glu Leu Tyr Pro Pro Tyr Phe Pro Ile Ser
            260                 265                 270

Gln Ala Ile Ala Glu Val Leu Val Asn Ser
        275                 280

<210>  9
<211>  177
<212>  PRT
<213>  Microcystis aeroginosa

<400>  9

Met Lys Ala Leu Glu Lys Leu Val Thr Glu Arg Leu Tyr Leu Val Pro
1               5                   10                  15

Leu Lys Leu Glu Leu Leu Lys Ala Ala Ile Ile Gly Ile Asp Glu Leu
            20                  25                  30

Ala Thr Ile Leu Gly Val Lys Val Ala Asp Asp Trp Ala Glu Gln Glu
        35                  40                  45

Leu Ile Gln Ala Phe Pro Asp Ile Ala Asn Ile Leu Leu Gln Phe Pro
    50                  55                  60

Leu Gln Asn Glu Trp Gly Trp Gly Ser Leu Ile Ile His Gln Ala Glu
65                  70                  75                  80

Asn Thr Leu Ile Gly His Val Met Ile Lys Ile Ile Pro Asp Val Thr
                85                  90                  95

Gly Leu Pro Thr Asp Ala Val Glu Ile Gly Tyr Gln Val Ala Pro Leu
            100                 105                 110

Tyr Arg Arg Gln Gly Tyr Ala Ser Glu Ala Thr Lys Ala Met Met Asp
        115                 120                 125

Trp Thr Leu Ser Gln Pro Gly Met Gln Thr Val Thr Ala Gly Cys Ala
    130                 135                 140

Pro Asp Asn Ile Ala Ser Lys Arg Val Leu Glu Lys Ile Gly Met Asp
145                 150                 155                 160

Leu Ile Glu Ala Arg Glu Lys Val Leu Ile Trp Lys Leu Ser Arg Val
                165                 170                 175

Cys

<210> 10
<211> 2069
<212> DNA
<213> Microcystis aeroginosa

<400> 10
atgcctcaat atactactaa acaagcaacg gaaaaccctg ttgtgtccga ccagtccgcc 60

aataatccct tttcactatt tggtcaattt tgggaaagcc taaaaattgt ggctcaaccc 120

tattggtatc caacggagtt aaatggacgt gcctttggag atgtgatcat ttcttgggga 180

atgtcagctt tgatcctttt aagtatcttg gcaacagtcg gggtagaagc ttttagtagc 240

tactggaatc gttatgtact cgatattatt attgaagata gagatctttc taaatatcta 300

aatacgcttt ggttatccag tttgttgatt ataggaacaa cggggttatt tgctttttct 360

caatttattc gtcgtcaagt ggcattggat tggtataagt ggttaaccaa acaaaccgtt 420

aaaaaatatc tcaatgatcg cgcttattat aacattgatt ttacctcagc tttaaaaaat 480

cccgatcaaa gattatctca agaaattgaa ccaattacga ccatgaccct aaggttgtta 540

attaccttcg tggaaaaagg gttgcaaatg attacttttg cgattattct ttggacaatt 600

tctcggcaaa ttgcggttta tctaataatt tatacaatcg caggaaattt catagcaatt 660

tatctaactc aagaattaaa caaaattaat caatcggagc ttaatcataa agctgattat 720
aattatgctt taacccatgt gcgtaaccac gccgaatcaa ttgctttttt tcagggagaa 780
gagcaggaag aaaaaatcat tgaagaccgt tttcaacggg ttattcaaag ctcggaaaaa 840
ctgatcaatt gggaacggtt taataatctt tttaatcgag gttatcagtc agcgattagt 900
gtattttcaa tgtttatttt gaccccgatg tttattaaaa acgaaatcga ttatggggaa 960
attagtcaag cgagtttatg ctgtttcctt ttttctaatg ccttgggaca attaattact 1020
gaatggggaa catcgggtaa agtttcaagt tatattgaac ggttagctac cttgacagat 1080
gccttaaaag ttgccagtga agagccgaaa aacttaagtc gtattaccac cctggaagat 1140
aatcgtctag cttttgaaaa ttttacccta caaactcccg attatgaaaa ggtaattgtg 1200
gaaaatttat cggtttctgt ccctttggga gaaagcttac tgattattgg ccccagtggt 1260
cgaggaaaaa gttccttgtt acgagcaatc gccggtttat ggaaagcagg tagtggtcgt 1320
ttagttcgtc ctcctttagc taaaatgtta tttttacccc aacgtcctta tattatcctt 1380
ggcactttac gccagcaatt actttatccc cataccaatg agcaggtgag cgatgaagaa 1440
ttagaaaaca tcttaaaaaa agtcaacctg caacatttat taactgacaa aaataccttg 1500
gataaacaag tcaattggga acagatttta tccctcggag aacaacaaag attagctttt 1560
gctcgactat taatcactaa acccactttt actatcttag atgaagcaac cagcgcttta 1620
gatttggcca atgaagccag cttatatcag caattacagg aaagtgaaac cacctttatt 1680
agtgttggtc atcgggaaag tttatttaat tatcatcgat gggtcttaga gttaacagaa 1740
aattcccatt ggcagctgtc aactgtagaa gattatcagc gcaaaaaagt gaataaccta 1800
gtgatgatgt caaaaaaatc agaaaatttt tcgtcggtaa aaatagagac tttgtctgag 1860
gttaaaactg agcatgatgc ggaaattgcc ggagtttctg aaatagcaga aggtttgtcc 1920
catcaacaaa tgcagtccct aactgattat tctttaagta acgtccggag tcgtgccagt 1980
ttaggtaaaa ttattactgc caaagatggt tttaattatc gttacgataa agacccaaaa 2040
accttaaaat gggtgagaat atagctaat 2069

<210> 11
<211> 687
<212> PRT
<213> Microcystis aeroginosa

<400> 11

Met Pro Gln Tyr Thr Thr Lys Gln Ala Thr Glu Asn Pro Val Val Ser
1               5                   10                  15

Asp Gln Ser Ala Asn Asn Pro Phe Ser Leu Phe Gly Gln Phe Trp Glu
            20                  25                  30

Ser Leu Lys Ile Val Ala Gln Pro Tyr Trp Tyr Pro Thr Glu Leu Asn
        35                  40                  45

Gly Arg Ala Phe Gly Asp Val Ile Ile Ser Trp Gly Met Ser Ala Leu
    50                  55                  60

Ile Leu Leu Ser Ile Leu Ala Thr Val Gly Val Glu Ala Phe Ser Ser
65                  70                  75                  80

Tyr Trp Asn Arg Tyr Val Leu Asp Ile Ile Ile Glu Asp Arg Asp Leu
                85                  90                  95

Ser Lys Tyr Leu Asn Thr Leu Trp Leu Ser Ser Leu Leu Ile Ile Gly
            100                 105                 110

Thr Thr Gly Leu Phe Ala Phe Ser Gln Phe Ile Arg Arg Gln Val Ala
        115                 120                 125

Leu Asp Trp Tyr Lys Trp Leu Thr Lys Gln Thr Val Lys Lys Tyr Leu
    130                 135                 140

Asn Asp Arg Ala Tyr Tyr Asn Ile Asp Phe Thr Ser Ala Leu Lys Asn
145                 150                 155                 160

Pro Asp Gln Arg Leu Ser Gln Glu Ile Glu Pro Ile Thr Thr Met Thr
                165                 170                 175

Leu Arg Leu Leu Ile Thr Phe Val Glu Lys Gly Leu Gln Met Ile Thr
            180                 185                 190

Phe Ala Ile Ile Leu Trp Thr Ile Ser Arg Gln Ile Ala Val Tyr Leu
        195                 200                 205

Ile Ile Tyr Thr Ile Ala Gly Asn Phe Ile Ala Ile Tyr Leu Thr Gln
    210                 215                 220

Glu Leu Asn Lys Ile Asn Gln Ser Glu Leu Asn His Lys Ala Asp Tyr
225                 230                 235                 240

Asn Tyr Ala Leu Thr His Val Arg Asn His Ala Glu Ser Ile Ala Phe
                245                 250                 255

Phe Gln Gly Glu Glu Gln Glu Glu Lys Ile Ile Glu Asp Arg Phe Gln
            260                 265                 270

Arg Val Ile Gln Ser Ser Glu Lys Leu Ile Asn Trp Glu Arg Phe Asn
        275                 280                 285

Asn Leu Phe Asn Arg Gly Tyr Gln Ser Ala Ile Ser Val Phe Ser Met
    290                 295                 300

Phe Ile Leu Thr Pro Met Phe Ile Lys Asn Glu Ile Asp Tyr Gly Glu
305                 310                 315                 320

Ile Ser Gln Ala Ser Leu Cys Cys Phe Leu Phe Ser Asn Ala Leu Gly
                325                 330                 335

Gln Leu Ile Thr Glu Trp Gly Thr Ser Gly Lys Val Ser Ser Tyr Ile
            340                 345                 350

Glu Arg Leu Ala Thr Leu Thr Asp Ala Leu Lys Val Ala Ser Glu Glu
        355                 360                 365

Pro Lys Asn Leu Ser Arg Ile Thr Thr Leu Glu Asp Asn Arg Leu Ala
    370                 375                 380

Phe Glu Asn Phe Thr Leu Gln Thr Pro Asp Tyr Glu Lys Val Ile Val
385                 390                 395                 400

Glu Asn Leu Ser Val Ser Val Pro Leu Gly Glu Ser Leu Leu Ile Ile
                405                 410                 415

Gly Pro Ser Gly Arg Gly Lys Ser Ser Leu Leu Arg Ala Ile Ala Gly
            420                 425                 430

Leu Trp Lys Ala Gly Ser Gly Arg Leu Val Arg Pro Pro Leu Ala Lys
        435                 440                 445

Met Leu Phe Leu Pro Gln Arg Pro Tyr Ile Ile Leu Gly Thr Leu Arg
    450                 455                 460

Gln Gln Leu Leu Tyr Pro His Thr Asn Glu Gln Val Ser Asp Glu Glu
465                 470                 475                 480

Leu Glu Asn Ile Leu Lys Lys Val Asn Leu Gln His Leu Leu Thr Asp
                485                 490                 495

Lys Asn Thr Leu Asp Lys Gln Val Asn Trp Glu Gln Ile Leu Ser Leu
            500                 505                 510

Gly Glu Gln Gln Arg Leu Ala Phe Ala Arg Leu Leu Ile Thr Lys Pro
        515                 520                 525

Thr Phe Thr Ile Leu Asp Glu Ala Thr Ser Ala Leu Asp Leu Ala Asn
    530                 535                 540

Glu Ala Ser Leu Tyr Gln Gln Leu Gln Glu Ser Glu Thr Thr Phe Ile
545                 550                 555                 560

Ser Val Gly His Arg Glu Ser Leu Phe Asn Tyr His Arg Trp Val Leu
                565                 570                 575

Glu Leu Thr Glu Asn Ser His Trp Gln Leu Ser Thr Val Glu Asp Tyr
            580                 585                 590

Gln Arg Lys Lys Val Asn Asn Leu Val Met Met Ser Lys Lys Ser Glu
        595                 600                 605

Asn Phe Ser Ser Val Lys Ile Glu Thr Leu Ser Glu Val Lys Thr Glu
    610                 615                 620

His Asp Ala Glu Ile Ala Gly Val Ser Glu Ile Ala Glu Gly Leu Ser
625                 630                 635                 640

His Gln Gln Met Gln Ser Leu Thr Asp Tyr Ser Leu Ser Asn Val Arg
                645                 650                 655

Ser Arg Ala Ser Leu Gly Lys Ile Ile Thr Ala Lys Asp Gly Phe Asn
            660                 665                 670

Tyr Arg Tyr Asp Lys Asp Pro Lys Thr Leu Lys Trp Val Arg Ile
        675                 680                 685

<210>  12
<211>  22
<212>  DNA
<213>  Nostoc 7120

<400>  12
gtgggatggc ttttgggaca gc                                              22

<210>  13
<211>  28
<212>  DNA
<213>  Nostoc 7120

<400>  13
gaatctcgat tgcttcggaa atggataa                                        28

<210>  14
<211>  14
<212>  PRT
<213>  Microcystis aeroginosa

<400>  14

Tyr Gly Gly Thr Phe Lys Tyr Pro Ser Asp Trp Glu Glu Tyr
1               5                   10

<210>  15
<211>  14
<212>  PRT
<213>  Microcystis aeroginosa

<400>  15

Phe Gly Thr Thr Leu Lys Tyr Pro Ser Asp Trp Glu Glu Tyr
1               5                   10

<210>  16
<211>  14
<212>  PRT

<213>  Microcystis aeroginosa

<400>  16

Phe Gly Thr Thr Leu Lys Tyr Pro Ser Asp Trp Glu Glu Tyr
1               5                   10


<210>  17
<211>  14
<212>  PRT
<213>  Microcystis aeroginosa

<400>  17

Phe Gly Asn Thr Met Lys Tyr Pro Ser Asp Trp Glu Glu Tyr
1               5                   10


<210>  18
<211>  13
<212>  PRT
<213>  Microcystis aeroginosa

<400>  18

Phe Ser Thr Tyr Lys Tyr Pro Ser Asp Phe Glu Asp Phe
1               5                   10


<210>  19
<211>  13
<212>  PRT
<213>  Microcystis aeroginosa

<400>  19

Phe Ser Thr Tyr Lys Tyr Pro Ser Asp Phe Glu Asp Phe
1               5                   10


<210>  20
<211>  14
<212>  PRT
<213>  Microcystis aeroginosa

<400>  20

Tyr Pro Gln Thr Leu Lys Tyr Pro Ser Asp Trp Glu Glu Tyr
1               5                   10


<210>  21
<211>  14
<212>  PRT
<213>  Microcystis aeroginosa

<400>  21

Tyr Pro Gln Thr Leu Lys Tyr Pro Ser Asp Trp Glu Glu Tyr
1               5                   10


<210>  22
<211>  13
<212>  PRT
<213>  Microcystis aeroginosa

<400>  22

Ile Ser Thr Arg Lys Tyr Pro Ser Asp Trp Glu Glu Trp
1               5                   10


<210>  23
<211>  14
<212>  PRT
<213>  Nostoc 7120

<400>  23

Lys Arg Gly Gln Thr Arg Lys Tyr Pro Ser Asp Cys Glu Asp
1               5                   10


<210>  24
<211>  14
<212>  PRT
<213>  Nostoc7120

<400>  24

Asp Ile Ala Val Thr Leu Lys Tyr Pro Ser Asp Asn Glu Asp
1               5                   10


<210>  25
<211>  14
<212>  PRT
<213>  Nostoc 7120

<400>  25

Glu Ile Val Thr Leu Lys Phe Pro Ser Asp Asp Asp Asp Gln
1               5                   10


<210>  26
<211>  14
<212>  PRT
<213>  Microcystis aeroginosa

<400>  26

Phe Gly Thr Thr Leu Lys Tyr Ala Ser Asp Trp Glu Ala Tyr
1               5                   10


<210>  27
<211>  13
<212>  PRT
<213>  Microcystis aeroginosa

<400>  27

Ile Ser Thr Arg Lys Tyr Pro Ser Asp Trp Glu Glu Tyr
1               5                   10

## Description

Many pharmaceutically active natural products are known that comprise amino acids as their main building blocks. Many of these compounds are not based solely on α-amino-peptidic bonds, by which proteins are constituted from amino acids, but exhibit bonds between amino acid building blocks that comprise esters, o-amide bonds and other non-peptide linkages.

One important class of amino-acid-derived natural products is the group of depsipeptides. In depsipeptides, in addition to α-amino peptidic bonds, ester bonds or isoamide bonds, or both, between amino acids or hydroxy acids bridge the amino acid chain. Naturally occurring depsipeptides are usually cyclic, often comprising more than one ring. Many known depsipeptides such as actinomycin and valinomycin show potent antibiotic or other pharmacological activity.

Different micro-organisms produce depsipeptides or other non-ribosomally derived natural products comprising non-strictly α-amino-peptide linkages. Cyanobacteria of the genus Microcystis, known from toxic algae blooms in lakes and ponds, produce a variety of pharmaceutically active or toxic small molecule compounds, such as microcystin, aeruginosin or microviridin.

The structure of most natural compounds derived from non-ribosomal synthesis is complex. Many such compounds are synthesized through multi-step biosynthetic pathways in their respective organisms of origin. The elucidation of such pathways has greatly contributed to the ability to produce complex natural compounds synthetically through biotechnological methods (Stachelhaus, Science 1995, 269, 69-72). For most compounds, however, no synthetic pathway is known.

The provision of means to produce complex natural molecules through biosynthesis in organisms amenable to manipulation is important both from economic and medical viewpoints, as these compounds often are highly active, but very difficult and expensive to obtain by traditional organic multi-step synthesis.

Among the pharmaceutically active compounds that can be obtained from cyanobacteria, cyanovirin and curacin A are currently undergoing clinical evaluation. Another group of compounds of high interests are microviridins. These are tricyclic depsipeptides that have a cage-like structure and inhibit enzymes such as proteases with high selectivity. Microviridin B, for example, inhibits elastase specifically, whereas microviridin J inhibits chymotrypsin. Because of their high selectivity of inhibition, these compounds lend themselves to possible clinical uses, as their selective mode of action promises fewer side effects.

The current assumption is that depsipeptide synthesis involves non-ribosomal pathways (Schwarzer et al., Nat Prod Rep 20: 275-278 (2003)). The synthesis of patellamides from cyanobacteria-derived expression cassettes in E. coli has been published recently (Schmidt et al., Nature Chemical Biology; doi:10.1038/nchembio829; WO 2007/103739). Patellamides are a class of α-amino-linked cyclic peptide compounds that are less complex than depsipeptides.

Departing from this state of the art, it is the objective of the present invention to provide means and methods to synthesize complex pharmaceutically active natural products comprising α-amino peptide bonds and non-α-amino peptide linkages by biotechnological production in micro-organisms. This objective is attained by the nucleic acid sequence, the cell and the method according to the independent claims.

During the course of our investigation into non-ribosomal or alternative ribosomal pathways of microviridin synthesis, we found several open reading frames from the cyanobacteria strain Nostoc sp. PCC7120 that comprise open reading frames putatively encoding the amino acid sequence KYPSD. This peptide sequence is a conserved sequence in microviridins. The finding came as a surprise, as the strain Nostoc PCC 7120 is not known to produce microviridins. After sequence analysis of the immediate vicinity of the sequence encoding the conserved microviridin peptide motive KYPSD, two candidate open reading frames (ORF) were identified that exhibit sequence similarity to enzymes with ATP-grasp-type ligase activity. Such enzymes are known to catalyse the formation of ester and/or amide bonds in peptide structures. An enzyme of the ATP-grasp-type ligase has been implicated in the biosynthesis of the natural compound marinostatin in Alteromonas, although no direct evidence was provided (Miyamoto et al., Biosci Biotechnol Biochem 62:2446-2449 (1998)).

One of the two ATP-grasp-type ligases discovered as part of the putative microviridin gene cluster shows close similarity (70%) to the ATP grasp ligase implicated in ester bond formation in marinostatin biosynthesis, whereas the second ATP grasp ligase encoded by the microviridin gene cluster presumably represents a new type of ATP grasp ligase facilitating amide bond formation, which is first described here for a natural product pathway. Both enzymes show only very low similarity to biochemically characterized ATP grasp type ligases such as the glutathione synthetases or the D-Ala-D-Ala ligase (<20%).

The surprising finding in the cyanobacteria strain Nostoc, of a genomic sequence putatively encoding a conserved peptide known from the microviridin family of depsipeptides, in close vicinity to ATP-grasp-type ligase encoding open reading frames, for the first time allowed the hypothesis that the microviridin family of natural compounds is based on a ribosome-synthesized precursor peptide that is modified by post-translational enzymatic reactions.

A fosmid library of the strain M. aeruginosa NIES298, which is known to synthesize microviridin, was screened for genes encoding potential microviridin encoding sequences with a DNA probe derived from sequences in the vicinity of the Nostoc KYPSD-containing ORF. This enabled the identification of a sequence cluster comprising an open reading frame potentially encoding a peptide precursor sequence matching the microviridin compound associated with that strain, microviridin B. Two ATP-grasp-type ligase open reading frames and one open reading frame potentially encoding an N-acetyltransferase enzyme was found in the immediate vicinity of the microviridin precursor sequence.

The nucleic acid sequence encoding the precursor peptide is given in Seq. ID no. 001. The sequences encoding ATP-grasp-type ligase enzymes are given in Seq. ID no. 002 and 003, respectively. The N-acetyltransferase type sequence is given in Seq. ID no. 004. The microviridin biosynthesis operon of the strain M. aeruginosa NIES298 is given in Seq. ID no. 005. The amino acid sequences of the respective encoded proteins are given in Seq Id Nos. 006 to 009.

According to one aspect of the present invention, a nucleic acid molecule is provided that comprises a nucleotide sequence encoding a precursor peptide molecule that can be post-translationally modified to yield a pharmaceutically active depsipeptide compound. Typically, such peptide sequences comprise 5 to 50 amino acids, preferably between 9 to 24 amino acids; most preferred 12 to 18 amino acids. According to one preferred aspect, such pharmaceutically active depsipeptide compound is a microviridin containing the KYPSD core amino acids, more preferred microviridin B or J. A nucleotide sequence encoding a peptide molecule that can be post-translationally modified to yield a pharmaceutically active depsipeptide compound can be the sequence of Seq. ID 001.

A precursor peptide is a sequence of amino acids linked by classic alpha-amidic peptide bonds that can be modified by post-translational modification. A depsipeptide precursor peptide is a sequence of amino acids linked by classic alpha-amidic peptide bonds that can be modified by post-translational modification to yield a depsipeptide. A microviridin precursor peptide is a sequence of amino acids linked by classic alpha-amidic peptide bonds that can be modified by post-translational modification to yield a microviridin.

Posttranslational modification as used in the preceding paragraph means any chemical modification to a peptide sequence of the 20 standard proteinogenic amino acids known to occur in cells; more specifically, the term posttranslational modification refers to the introduction of ester bonds and isoamide bonds within a chain of amino acids linked by alpha-amidic peptide bonds.

Among the Microviridin precursor peptide sequences that can be used according to the present invention are YGGTFKYPSDWEEY, GTTLKYPSDWEEY, FGNTMKYPSDWEEY, FSTYKYPSDFEDF, YPQTLKYPSDWEEY, ISTRKYPSDWEEW, KRGQTRKYPSDCED, DIAVTLKYPSDNED, EIVTLKFPSDDDDQ. These sequences are the prepeptide sequences found in the depsipeptide molecule. Typically, signal sequences will precede these peptide sequences. One example is found in the MdnA sequence (Seq ID No 001 and Seq ID No 006).

According to one aspect of the current invention, the nucleic acid sequence encoding a precursor peptide encodes a precursor peptide for microviridin B or microviridin J.

According to another aspect of the current invention, a nucleic acid sequence is provided, encoding enzymes for the post-translational modification of a depsipeptide precursor peptide. Preferably, the sequences encoding precursor-peptide modifying enzymes additionally encode ATP-grasp-type ligase and/or N-acetyltransferase type enzymes. More preferably, the enzymes additionally encoded by these sequences catalyze the formation of ester or isoamide bonds between amino acid residues in a peptide chain.

According to one preferred aspect of the invention, the sequences encoding precursor-peptide modifying enzymes comprise the sequences of Seq. ID 002, 003 and /or 004, or encode sequences of at least 80% identity, preferably 85% identity, most preferred 90 or 95% identity, and with substantially similar function as regards the encoded proteins, to sequences Seq. ID No 6, 7, 8, 9, 11.

According to one preferred embodiment of the present invention, an inventive nucleotide sequence furthermore can comprise a sequence element MdnE. MdnE is an ABC-type transporter that shows similarity (∼72%) to other peptide transporters in cyanobacteria such as McyH and McnF that are implicated in transport of microcystin and cyanopeptolin, respectively. The amino acid sequence of MdnE and the nucleic acid sequence encoding it are shown in Seq Id Nos 11 and 10, respectively.

According to yet another aspect of the current invention, a sequence is provided comprising sequences encoding one or more nucleic acid molecules encoding a peptide molecule that can be post-translationally modified to yield a pharmaceutically active depsipeptide compound, and nucleic acid sequences that encode one or more enzymes for the post-translational modification of a depsipeptide precursor peptide, preferably precursor-peptide modifying enzymes such as ATP-grasp-type ligase and/or N-acetyltransferase type enzymes. According to one preferred embodiment of this aspect of the invention, a sequence is provided comprising sequences encoding a microviridin precursor peptide, at least one and preferably two ATP-grasp-type ligase enzymes and a N-acetyltransferase. One example of such sequence is Seq. ID no. 005.

According to another aspect of the current invention, a cell is provided that contains one ore more nucleic acid sequences as specified in the preceding paragraphs, namely, sequences encoding one or more nucleic acid molecules encoding a peptide molecule that can be post-translationally modified to yield a pharmaceutically active depsipeptide compound, and /or nucleic acid sequences that encode one or more enzymes for the post-translational modification of a depsipeptide precursor peptide, preferably precursor-peptide modifying enzymes such as ATP-grasp-type ligase and/or N-acetyltransferase type enzymes. Cells are preferred for the manipulation of which by genetic methods protocols are established. Prokaryotic cells of the genera E. coli and Bacillus subtilis are especially preferred.

According to yet another aspect of the current invention, a method for the production of pharmaceutically active depsipeptide natural products is provided, by which cells comprising nucleic acid sequences specified in the preceding paragraphs are grown in a suitable medium, and a pharmaceutically active depsipeptide compound is harvested from the medium or the cells, purified by suitable methods and provided for the use as a pharmaceutically active compound.

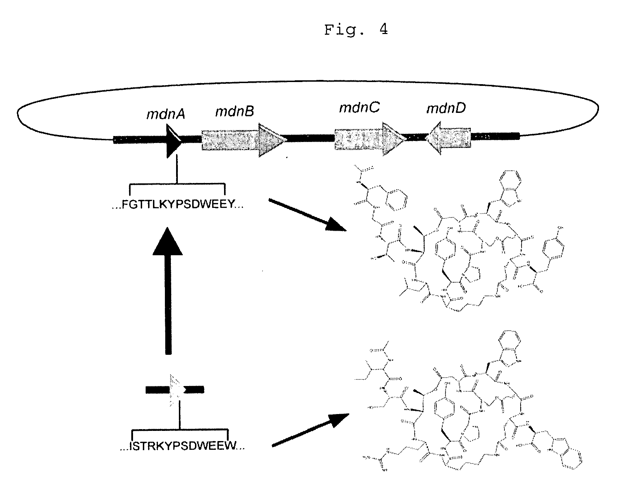

According to yet another aspect of the current invention, a general method is provided for the production of depsipeptide natural products of great sequence variety by a simple and versatile process. According to this aspect of the invention, the precursor gene mdnA of the mdn gene cluster is replaced with a precursor carrying a customized microviridin coding sequence. By variation of the precursor sequence, substantial variation of the resultant peptide precursor and finally produced product can be attained by replacing single amino acid residues, short sequences of amino acids or the entire sequence. Such substitution can be applied to any of the positions, or positions can be added. By parallel examination of sequence permutations, high numbers of potential drug candidates may be produced and screened. The principle of the process is depicted in Fig. 4.

Examples for manipulated precursor sequences that can be obtained by such pathway are:
FGTTLKYASDWEAY (Point mutated variant of Microviridin B with altered KYPSD motif) and ISTRKYPSDWEEY (shuffled variant of Microviridin B and J).

Replacement of the precursor sequence, or parts thereof, can be attained by recombination, insertion of artificially synthesized oligomers or other methods known in the art.

The invention is described in further detail in the following Figures and examples.

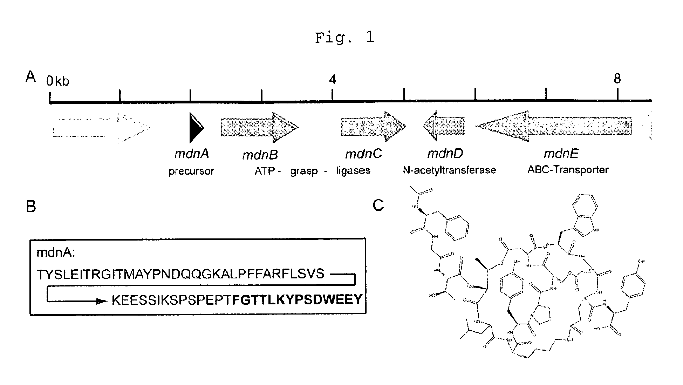

- Fig.1: shows the biosynthesis of microviridin in M. aeruginosa NIES298; in particular A) the organization of the mdn microviridin biosynthetic gene cluster in M. aeruginosa NIES298. Biosynthesis and transporter genes are indicated in grey. The precursor protein is shown in black. The light grey arrows represent open reading frames adjacent to the mdn gene cluster. B) the MdnA sequence. The bold type sequences show the product coding sequence. C) the chemical structure of MicroviridinB.

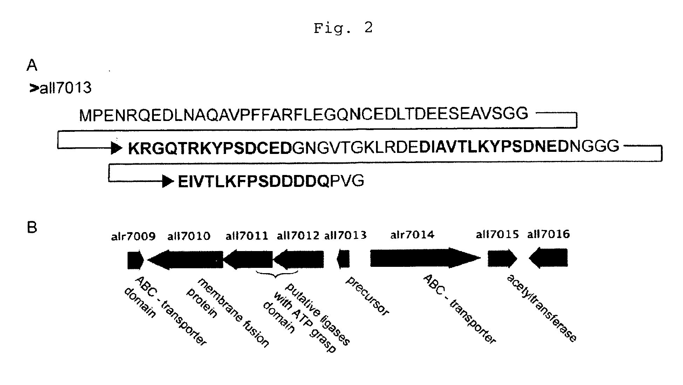

- Fig.2: shows the putative microviridin genes in Nostoc PCC7120 A) the protein sequence of all7013. The bold type sequences show the putative microviridin coding sequence. B) the organization of ORFs in the vicinity of all7013, including two ATP grasp ligase homologues, an acetyltransferase and an ABC-transporter homologue.

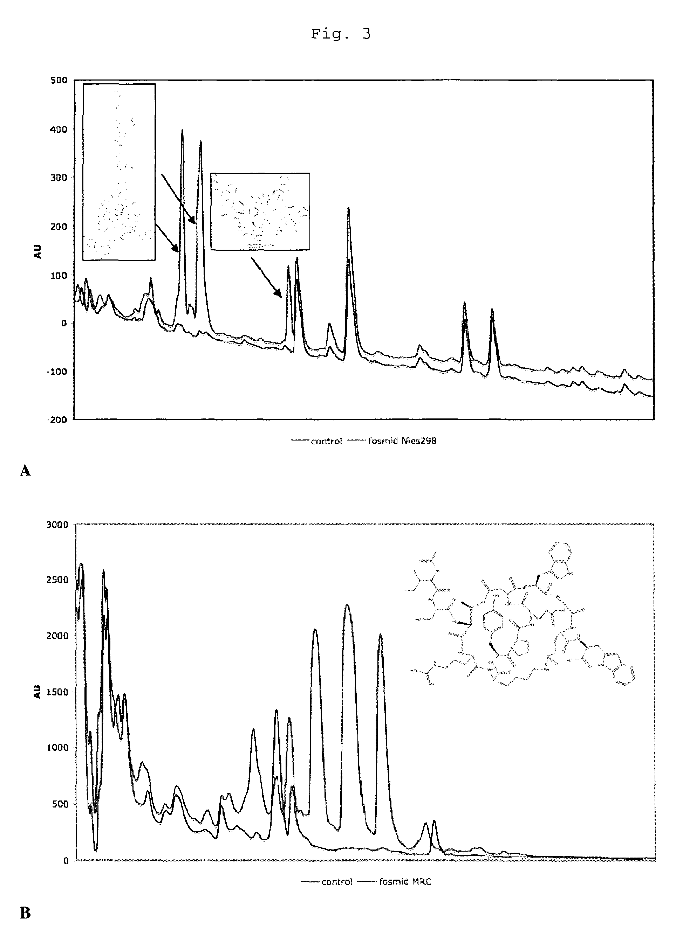

- Fig.3: shows HPLC chromatograms of A) extracts from E. coli strains harboring a fosmid either carrying (grey, upper baseline) or lacking (black, lower baseline) the mdn gene cluster from M. aeruginosa NIES298. Arrows indicate two peaks representing two microviridin-like structures and one peak corresponding to microviridin B, and B) HPLC Profiles of extracts from E. coli strains harboring a fosmid either carrying (grey, upper baseline) or lacking (black, lower baseline) the mdn gene cluster from M. aeruginosa MRC. Numbers 1 to 3 correspond to currently unknown substances showing structural similarity to microviridin J (4).
- Fig.4: shows a general strategy for manipulation and expression of different microviridin like substances

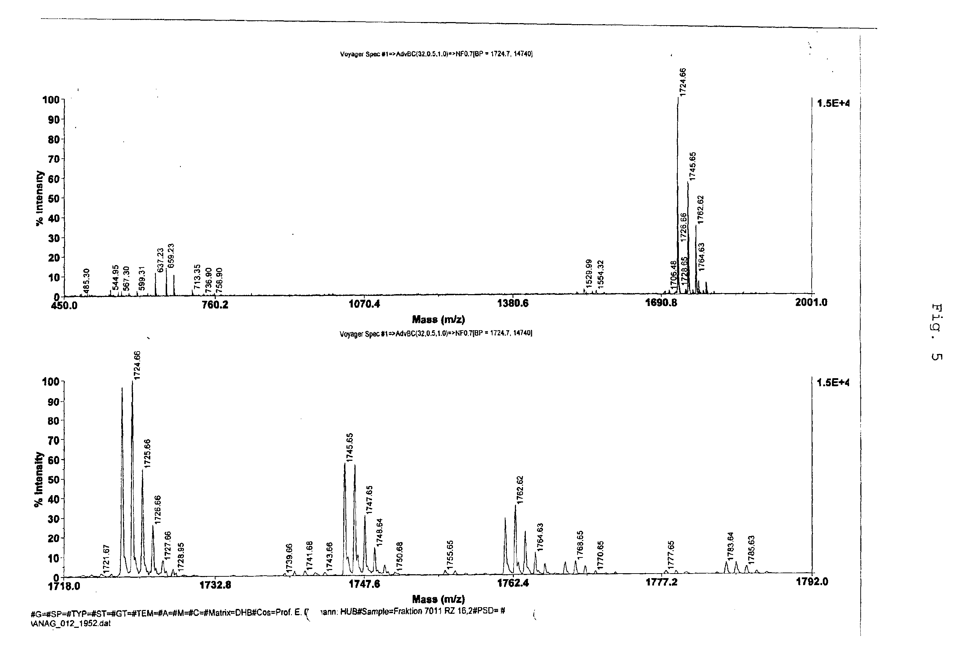

- Fig. 5: shows a MALDI-TOF spectrum of the peak corresponding to Microviridin B shown in Fig. 3A. The upper spectrum shows the spectrum obtained by standard MALDI procedure, the lower spectrum a fragmentation run rendering the fragmentation pattern expected from Microviridin B. The mass of 1724 corresponds to Microviridin B.

### Examples

### Fosmid Library Construction and Screening

Genomic DNA was prepared from M. aeruginosa NIES298 and MRC as reported previously (Franche, Damerval (1988). Methods in Enzymology). DNA fragments of approximately 30 to 40kb were directly ligated to the pCC1 FOS vector (Epicentre Technologies, USA) following manufacturer instructions. Screening of the library was performed by hybridization using standard conditions (Sambrook et al., 1989; Molecular cloning. A laboratory manual; Cold Spring Harbour Lab Press). The all7012 gene fragment used as a probe was obtained by PCR using the primer 7012fw 5'-GTGGGATGGCTTTTGGGACAGC-3' and 7012rev 5'-GAATCTCGATTGCTTCGGAAATGGATAA-3'. The DNA probe was radioactively labeled with the HexaLabel Kit (Fermentas, Germany).

### Extraction and Isolation

After identification of fosmid clones containing the mdn gene cluster, a total volume of 100ml of each E. coli clone was induced to high copy number according to the suggestions of the manufacturer (Epicentre Technologies, USA). The harvested cells were resuspended in deionized water and lysed by sonication. The cell debris was removed by centrifugation. The resulting supernatant was loaded on a Sep-Pak cartridge (Waters Corporation, USA), which then was washed with 5% methanol. The components of interest were eluted with 100% methanol. Extracts were subjected to reversed phase column HPLC (Symmetrie Shield RP18 3,5mm, 20-80% aquaeous acetonitril containing 0,1% TFA, flow rate 1ml/min, UV detection at 210nm).

### DNA Sequencing and Sequence Analysis

DNA sequencing using primer walking strategy was performed by SMB GbR (Berlin, Germany). The assembly was performed using the software package Vector NTI (Invitrogen, Germany). Analyses of DNA sequences and deduced protein sequences were performed using the NCBI (National Institute for Biotechnology Information, Bethesda, MD) BLAST server (http://www.ncbi.nlm.nih.gov/blast/).

## Claims

1. A nucleic acid molecule comprising
- a nucleic acid sequence encoding a precursor peptide of between 5 and 50 amino acids, which is susceptible to post-translational modification,
- one or more nucleic acid sequences encoding enzymes for post-translational modification, of which at least one is an ATP-grasp-like ligase enzyme.

2. A nucleic acid molecule according to claim 1, wherein the nucleic acid sequences encoding a precursor peptide and encoding enzymes are under the control of promoters operable in prokaryotes.

3. A nucleic acid molecule according to at least one of claim 1 or 2, wherein the nucleic acid sequences encoding a precursor peptide and encoding enzymes are under the control of promoters operable in Bacillus, E. coli or Microcystis.

4. A nucleic acid sequence according to at least one of claims 1 to 3, wherein the nucleic acid sequence encoding a precursor peptide encodes a precursor peptide of between 9 and 24 amino acids.

5. A nucleic acid molecule according to at least one of claims 1 to 4, wherein the precursor peptide is a depsipeptide precursor.

6. A nucleic acid molecule according to claim 5, wherein the precursor peptide is a microviridin precursor peptide.

7. A nucleic acid molecule according to at least one of claims 1 to 6, wherein the one or more nucleic acid sequences encoding enzymes for post-translational modification encode at least one N-acetyltransferase.

8. A nucleic acid molecule according to at least one of claims 1 to 7, wherein the one or more nucleic acid sequences encoding enzymes for post-translational modification encode at least one ABC-type transporter protein.

9. A nucleic acid molecule according to at least one of claims 1 to 8, comprising at least one of the sequences Seq. ID No 1, 2, 3, 4, 5, or 10, or a sequence encoding at least one polypeptide at least 80% identical and functionally homologous to Seq. ID No 6, 7, 8, 9, or 11.

10. A nucleic acid molecule according to at least one of claims 1 to 9, comprising a nucleic acid sequence encoding at least one of the peptide sequence Seq. ID 6, 14, 15, 16, 17, 18, 19, 20, 21, 22, 23, 24, 25, 26, or 27.

11. Cell comprising a nucleic acid molecule according to at least one of claims 1 to 10.

12. Cell according to claim 11, wherein the cell is a prokaryote.

13. Cell according to claim 12, wherein the cell is a Bacillus, E. coli or Microcystis cell.

14. Method for the synthesis of a depsipeptide, comprising the steps of growing a cell according to at least one of claims 11 to 13 in a suitable medium and isolating a compound obtained by post-translational modification of the precursor peptide.
